# EUROPEAN PATENT APPLICATION

(11) **EP 1 393 734 A1**
(43) Date of publication of application: **03.03.2004**
(21) Application number: 02728122.9
(22) Date of filing: 23.05.2002
(51) Int. Cl.: A61K 31/716, A61K 35/84, A61P 1/00, A61P 29/00

(54) **DRUGS FOR INTESTINAL DISEASES**

(30) Priority: 01.06.2001 JP 2001166982
(71) Applicant: Ajinomoto Co., Inc., Tokyo 104-8315 (JP)
(72) Inventor: MURATA, Yukie, Kawasaki-shi, Kanagawa 210-0801 (JP); HAMURO, Junji, Kawasaki-shi, Kanagawa 210-0801 (JP)
(74) Representative: HOFFMANN EITLE
(86) International application number: PCT/JP2002/004980
(87) International publication number: WO 2002/098433

(57) **Abstract**

Provides a drug product for the prevention, improvement in conditions relating to, and/or treatment of intestinal disease, characterized by comprising an active component in the form of β (1→3) glucan derived from vegetable material and having a molecular weight of from 5,000 to 20,000. Can be widely employed as a drug product for various intestinal diseases, particularly inflammatory bowel disease. Has almost no side effects, is highly effective when orally administered, and can be conveniently employed by numerous patients. Further, can be employed in the form of a health food product.

Also provides a method of preventing, improving conditions relating to, and/or treating intestinal disease and the use of the above-specified glucan as an active component in drug products.

## Description

### Technical Field

The present invention relates to a novel drug product specifically suited to the prevention, improvement in conditions relating to, and/or treatment of inflammatory bowel disease. It is highly safe, may be administered orally, and may thus be conveniently applied to a broad range of patients. The present invention further relates to a method of treating, improving conditions relating to, and/or preventing intestinal disease, and the use of the specific active components employed in this drug product in drug products for the prevention, improvement in conditions relating to, and/or treatment of intestinal disease.

### Prior Art

Intestinal disease, particularly inflammatory bowel disease, is a disease with a growing number of victims for which the necessity of prevention and treatment has been increasing in recent years. The pervasiveness of intestinal bacteria and a compromised immune system are the assumed causes of this disease. However, the cause of onset is not clearly any one morbidity increasing mechanism. There is a great deal of difference in morbidity between ulcerative colitis and Crohn's disease, which are typical inflammatory intestinal diseases. In the former, the contribution of stimulated humoral immunity exacerbation is high, while in the latter, while stimulated cell-mediated immunity exacerbation is high, the details are unknown. Accordingly, the means of preventing this disease and improving morbidity are limited and unsatisfactory. Particularly from the perspective of prevention, nothing has been provided that satisfies the needs of the treatment domain.

### Problems to Be Solved by the Invention

Given the above-described situation, there is a need for the development of a drug product for intestinal disease, particularly a drug product, and food product, that can be widely employed for inflammatory bowel disease, that have almost no side effects, are effective when orally administered, and that can be conveniently employed by many patients. The problem solved by the present invention is the development of such a drug product and preventive food product.

### Disclosure of the Invention

The present inventors conducted extensive research into solving the above-stated problem. They discovered that in model animal experiments on mice given drinking water containing β (1→3) glucan with a specific molecular weight derived from vegetable material, the above-described desirable pharmacological effects were achieved. They discovered that this specific glucan could be used as a drug product for preventing, improving conditions relating to, and treating intestinal disease. The present invention was devised on the basis of these discoveries.

That is, the present invention is a drug product for the prevention, improvement in conditions relating to, and/or treatment of intestinal disease, characterized by comprising an active component in the form of β (1→3) glucan derived from vegetable material and having a molecular weight of from 5,000 to 20,000.

A further form of the present invention is a method of preventing, improving conditions relating to, and/or treating intestinal disease characterized in that β (1→3) glucan derived from vegetable material and having a molecular weight of from 5,000 to 20,000 is ingested by or administered to the body. The various above-described forms of the drug of the present invention may be employed in this administration or ingestion.

And a still further form of the present invention is the use of β (1→3) glucan derived from vegetable material and having a molecular weight of from 5,000 to 20,000 as a drug product for the prevention, improvement in conditions relating to, and/or treatment of intestinal disease. The various above-described forms of the drug product of the present invention may be employed as the drug product used in the prevention, improvement in conditions relating to, and/or treatment of intestinal disease.

### Brief Description of the Figures

[Fig. 1]

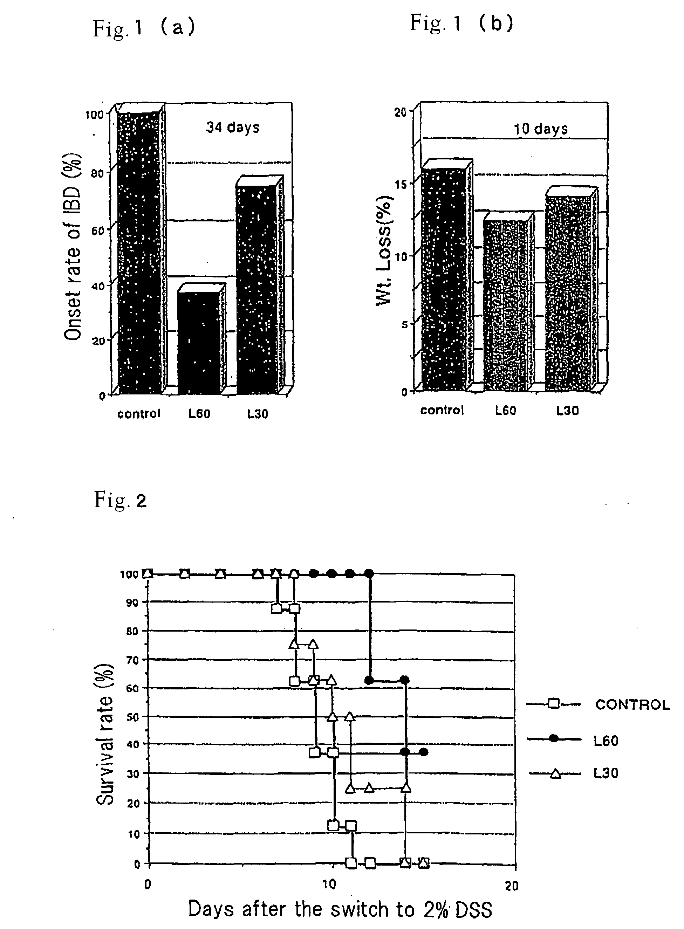

   Fig. 1a shows the onset rate (%) (Day 34 of switch to DSS 1 % aqueous solution) of inflammatory intestinal disease (IBD) in Embodiment 1.
   Fig. 1b shows the body weight reduction rate (%) (Day 10 of switch to DSS 1 % aqueous solution) in Embodiment 1.
   In Figs. 1a and 1b, the X-axis denotes, sequentially from the left, a control, Dry L60-min formic acid decomposed product, and Dry L30-min formic acid decomposed product.
[Fig. 2]
   Fig. 2 shows the survival rate (%) after the switch to DSS 2 % aqueous solution in Embodiment 1.
   □: Control; •: Dry L60-min formic acid decomposed product; Δ: Dry L30-min formic acid decomposed product.
[Fig. 3]

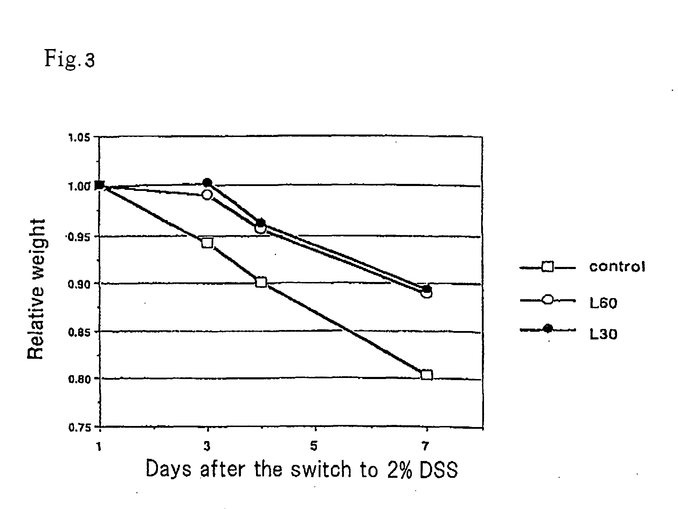

   Fig. 3 shows the change in body weight following the switch to DSS 2 % aqueous solution in Embodiment 1.
   Y-axis: The ratio when the weight at the start of the switch to DSS 2 % aqueous solution is taken as 1.
   □: Control; ○: Dry L60-min formic acid decomposed product; •: Dry L30-min formic acid decomposed product.

### Modes of Implementing the Invention

### Modes of implementing the invention are described below.

The application of the drug product of the present invention is not specifically limited beyond the prevention, improvement in conditions relating to, and treatment of intestinal disease, particularly inflammatory bowel disease. It is applied to mammals, usually humans (patients).

The intestinal disease to which the drug product of the present invention is applied is not specifically limited; it is particularly effective against inflammatory bowel disease typified by Crohn's disease and ulcerative colitis.

The active component employed in the drug product of the present invention is β (1→3) glucan derived from vegetable material and having a molecular weight of from 5,000 to 20,000. The use of a component having a molecular weight denoted as an average molecular weight of from 5,000-20,000 as the principal component is convenient. To prepare a glucan with a molecular weight falling within this range, a glucan of relatively high molecular weight that has been hydrolyzed to obtain a molecule of low molecular weight ― for example, degradated with an enzyme such as β-(1-3) glucanase, chemically decomposed with formic acid or the like, or degradated through the use of a physical method ― can be employed.

In the present invention, the term "β(1→3) glucan" includes all glucans having a β (1-3) bond, as well as glucans having a main chain in the form of a β(1→3) glusoside.

β (1→3) glucans obtained from mushrooms such as Matsutake [*Tricholoma matsudake*], Shiitake [*Lentinus edodes*], Bukuryo [*Poria cocos*], Kawaratake [*Coriolus versicolor*], Enokidake [*Flammulina veltipes*], Hiratake [*Pleurotus ostreatus*], Yamabushitake [*Hericium erinaceum*], and Agarikusuku [*Agaricus blazei murrill*] can be employed as the β (1→3) glucan derived from vegetable material. Such components can be readily prepared from mushrooms by, for example, obtaining an aqueous (hot water) extract, followed by precipitating with an alcohol (ethanol or the like) and, when needed, reducing the molecular weight thereof. Examples of methods for obtaining a molecular weight falling within the above-stated range are hydrolysis by suitable methods (enzymatic decomposition, hydrolysis with an acid such as formic acid, and decomposition by physical methods) (see Sasaki et al., Gann, 67, 191-195, April, 1976.).

One of characteristics of the drug product of the present invention is orally effective and also can be applied to treat IDDM (non insulin dependent diabetes). Safety is afforded, and there is no effect with regard to obesity. Accordingly, the form of administration is not specifically limited. Various forms of administration are possible, including oral administration and non-oral administration (intravenous administration and the like) are possible. Convenient, broad treatment of patients suffering from inflammatory bowel disease is possible. Due to safety and the suitability to oral administration, the drug product of the present invention can be employed in the form of health food products and therapeutic food products to prevention and improvement in such patients, as well as to patients having diseased intestines. It can also be widely applied in a preventative, therapeutic, and convenient fashion to patients who are at high risk for these diseases.

The present invention also permits mixing and combining with other drug product components (medically active substances). In such cases, so long as the active component of the present invention is present and the above-described targeted pharmacological activity is exhibited, the product is covered by the drug product of the present invention.

The incorporation of various substances that are pharmacologically acceptable in formulations (adjuvants and the like) is also possible. Formulation-use substances may be suitably selected based on the form of formulation. Examples are excipients, diluting agents, additives, anticaking agents, binders, coatings, lubricants, slipping agents, gloss-imparting agents, flavoring agents, sweetening agents, and solubilizing agents. Specific examples of formulation-use substances are magnesium carbonate, titanium dioxide, lactose, mannitol, other sugars, talc, milk protein, gelatin, starch, cellulose and its derivatives, animal and plant oils, polyethylene glycol, and solvents such as sterile water and monohydric and polyhydric alcohols such as glycerol.

The drug product of the present invention can be prepared in the above-described known forms as well as various medical drug formulations to be discovered in the future for, for example, oral administration, intraperitoneal administration, cutaneous administration, and inhalation. Known methods and methods developed in the future can be suitably employed to prepare various forms of medical drug formulations of the drug product of the present invention.

Examples of these forms of medical drug formulations are suitable solid and liquid formulations, such as grains, powders, coated tablets, tablets, (micro)capsules, suppositories, syrups, juices, suspensions, emulsions, titrations, injection solutions, and formulations affording extended release of active substances.

It is necessary that the above-described component be incorporated in a quantity suitably large to exhibit its drug effect in the formulation of the present invention based on the above-listed formulations.

The dosage of the drug product of the present invention is suitably selected based on the severity and type of symptoms presented by the intestinal disease patient, the type of formulation, and the like. For example, in oral administration, based on the net weight of the active component, a daily dosage per patient of about 10 mg to 10 g is desirable, about 100 mg to 5 g is preferred, and about 500 to 2,000 mg is even more preferred. In severe cases, even larger doses are possible. In terms of administration frequency and intervals, one administration every few days or one administration a day are both possible. Usually, however, there are several administrations per day, perhaps divided into 2 to 4 administrations, preferably before meals. Further, when administered intravenously, a dosage of about one-tenth to one-twentieth that of the above-described oral administration dosage is sufficient.

The drug product of the present invention can be broadly applied preventively and amelioratively to combat intestinal disease, and to patients who have already suffered from such diseases, as a health food product, therapeutic food product, or special health food product to provide meals. When employed as a health food product or the like, the above-described orally administered formulation can be referred to and orally administrable components and additives required by health food products can be added in the preparation. In that case, the drug product of the present invention can be provided in the form of food products (including all items placed in the mouth and chewed, such as chewing gum, toothpaste), nutrition agents, infusion formulations, and the like. These are also covered by the use of the drug product of the present invention. Therapeutic food products may be in any form, including solids and liquids.

The mechanism by which the low molecular weight β (1→3) glucan of the present invention acts on intestinal disease is presumed to be as follows. β (1→3) glucan is thought to mainly target macrophages among inflammatory cells. The well-known impregnation by macrophages of the mucous membranes of the intestines is observed in inflammatory bowel disease, and these macrophages are thought to intensify the immune response to bacteria in the intestines. β (1→3) glucan is thought to act on the macrophages, suppressing their immune response intensifying action.

As set forth above, a further mode of the present invention is a method of preventing, improving conditions relating to, and/or treating intestinal disease characterized in that β (1→3) glucan derived from vegetable material and having a molecular weight of from 5,000 to 20,000 is ingested by or administered to the body, and a still further mode is the use of β (1→3) glucan derived from vegetable material and having a molecular weight of from 5,000 to 20,000 as a drug product for the prevention, improvement in conditions relating to, and/or treatment of intestinal disease.

All of these modes of the present invention, including their particular ingestion, administration, and use forms, can be readily practiced based on the above-described description of the drug product of the present invention, the embodiments described further below, and, as necessary, with reference to prior art.

### Desirable Modes of Implementing the Invention

The present invention is described in detail below based on embodiments. However, the present invention is not limited to the embodiments. The percentages employed in the embodiments are weight percentages unless specifically stated otherwise. (Samples and Laboratory Animals)
1) Dextran sodium sulfate; made by ICN Co., "Cat. 160110"
2) 13-Week C57/BL6 female mice (prepared by Japan Charles River)

### (Preparation of Mushroom Product Extracted with Hot Water and Decomposed with Formic Acid)

### 1) 60-Minute Hydrolysis Product

To a two-liter, round-bottom three-necked flask equipped with reflux condenser, temperature gauge, and mechanical stirrer were weighed out and charged 31.0 g of Dry L product, 620 mL of 80 % formic acid was added, and the mixture was placed in an oil bath preheated to 100°C. At 20 min, the temperature of the reaction solution reached 90°C, and stirring was continued for another 60 min. Subsequently, the reaction vessel was placed with being stirred in an ice water bath to stop the reaction. The reaction solution was cooled to room temperature, yielding a dark-brown gel-like substance. This was transferred to a two-liter eggplant-shaped flask. The solvent was evaporated under vacuum, yielding 50.4 g of a glue-like solid. To this were added two liters of pure water, the mixture was heated in a water bath to 60°C, the solid was dissolved to the extent possible, the mixture was left standing, and the clear supernatant was separated by decantation. To the undissolved portion in which was present a fibrous solid were added 800 mL of pure water and a household blender was employed to mix and pulverize for 60 min. To this was added the above-mentioned supernatant and the mixture was processed in an ultrasonic bath with ultrasound, yielding a uniform suspension. The suspension obtained was rapidly frozen in a dry-ice - alcohol bath and then freeze-dried, yielding 30.4 g of solid. A 0.22 % (by weight) suspension of this solid had a pH of 4.51. The average molecular weight was 12,200.

The Dry L consisted of 1 kg of raw Shiitake (*Lentinus edodes*) dissolved in 5,000 mL of hot water and then precipitated (320 g) from 5,000 mL of ethanol.

### 2) 30-Min Hydrolysis Product

The same operation as above was conducted with the exception that the period of heating after reaching 90°C was shortened to 30 min in the above-described method of preparing a 60 min hydrolysis product, yielding 30.8 g of solid. The 0.22 % (by weight) suspension thereof had a pH of 4.86. The average molecular weight was 25,000.

### (Preparation of an Enzyme Decomposition Product of the Hot Water Extract of Mushroom)

To a two-liter, round-bottom three-necked flask equipped with reflux condenser, temperature gauge, and mechanical stirrer were weighed out and charged 30.0 g of raw Shiitake hot-water extract. This was dissolved in 2 L of sterile water, commercial β-(1-3) glucanase was added, and the mixture was stirred at 30°C and reacted. The reaction continued for 60 min. Subsequently, the reaction vessel was immersed in an ice water bath to stop the reaction. The reaction solution was transferred to a five-liter eggplant-shaped flask, the solvent was distilled off under vacuum, and distillation was stopped before solid precipitated. The clear supernatant was separated and processed in an ultrasonic bath with ultrasound, yielding a uniform, clear solution. The solution obtained was quickly frozen in a dry-ice - alcohol bath and then freeze dried, yielding 32.4 g of solid. A 0.22 % (by weight) suspension of the solid had a pH of 6.52. The average molecular weight was 9,200.

### (Preparation of 0.02 Weight Percent Aqueous Solution of the Dry L Hydrolysis Product)

2 g quantities of Dry L60-min formic acid hydrolysis product and Dry L30-min formic acid hydrolysis product were dissolved in one-liter quantities of sterile water (double distilled, followed by filtration with a millipore filter) and the pH values thereof were measured. The pH values were then adjusted to close to pH 7.0 with 1 normal sodium hydroxide aqueous solution to prepare Dry L 0.02 weight % aqueous solutions.

### (Preparation of 0.1 Weight Percent Aqueous Solution of Dry L)

1 g of Dry L60-min formic acid decomposed product was dissolved in one liter of sterile water and the pH was measured. The pH was adjusted to close to 7.0 with a 1 normal sodium hydroxide aqueous solution to prepare a 0.1 weight % aqueous solution of Dry L.

### (Preparation of 1 Percent DSS Aqueous Solution)

10 g of dextran sulfate sodium was dissolved in one liter of sterile water to prepare 1 % DSS aqueous solution.

### (Embodiment 1)

### Test of Effect of Orally Ingested Dry L with Inflammatory Bowel Disease (DSS-Induced Inflammatory Bowel Disease) Model

Suppression of DSS-induced inflammatory bowel disease by the oral administration of Dry L decomposed product was confirmed. This is specifically described below.

The system where mice are made to drink an aqueous solution of dextran sulfate sodium (abbreviated to "DSS" hereinafter) is a known inflammatory bowel disease (abbreviated to "IBD" hereinafter) model. We employed this system to test whether the preadministration of an aqueous solution of Dry L60-min formic acid decomposed product would suppress or accelerate onset.

When IBD is induced, body weight decreased. Bloody feces, weight reduction, and the survival rate were employed as indicators of onset.

Female 13-week C57/BL6 mice were given drinking water in the form of an aqueous solution of Dry L60-min formic acid decomposed product and an aqueous solution of Dry L30-min formic acid decomposed product and a control group was given sterile water. On day 10 after the start of drinking water, the drinking water was switched to a DSS 1 % aqueous solution. Body weight was then measured once a week and subsequent survival was tracked.

The diminution in body weight on day 10 following the switch to DSS 1 % aqueous solution was 16 % in the control group and 12.5 % in the group given drinking water containing Dry L60-min formic acid decomposed product. A significant decrease in the group given drinking water containing L60-min formic acid decomposed product was confirmed (see Fig. 1b). The rate of onset (bloody feces) on day 34 following the switch to DSS 1 % aqueous solution was 100 % in the control group and 37 % in the group given drinking water containing Dry L60-min formic acid decomposed product. Significant suppression of onset was confirmed in the group given drinking water containing Dry L60-min formic acid decomposed product. Further, in the group given drinking water containing Dry L30-min formic acid decomposed product, 75 % onset rate %was confirmed as suppression of onset (see Fig. 1a).

On day 34 following the switch to DSS 1 % aqueous solution, a switch was made to DSS 2 % aqueous solution. On day 11 after the switch to DSS 2 % aqueous solution, the survival rate was 0 % in the control group and 100 % in the group given drinking water containing Dry L60-min formic acid decomposed product. Clear suppression of onset was confirmed in the group given drinking water containing L60-min formic acid decomposed product (see Fig. 2). On day 7 following the switch to DSS 2 % aqueous solution, the change in body weight, relative to a body weight of 1 at the time of the initial switch, was 0.8 in the control group and 0.9 in the group given drinking water containing L60-min formic acid decomposed product. The reduction in body weight of the group given drinking water containing Dry L60-min formic acid decomposed product was thus less than that in the control group, confirming the suppression of onset of IBD (see Fig. 3).

As is clear from the above results, the ingestion of drinking water containing Dry L60-min formic acid decomposed product was confirmed to significantly suppress inflammation in the IBD model.

### (Embodiment 2)

### Test of Effect of Orally Ingested Enzymatic Decomposition Product with Inflammatory Intestinal Disease (DSS-Induced Inflammatory bowel Disease) Model

Suppression of DSS-induced inflammatory bowel disease by the oral ingestion of enzymatic decomposition product of Shiitake hot-water extract was confirmed. This is specifically described below.

The system where mice are made to drink an aqueous solution of dextran sulfate sodium (abbreviated to "DSS" hereinafter) is a known inflammatory bowel disease (abbreviated to "IBD" hereinafter) model. We employed this system to test whether the preadministration of an aqueous solution of Shiitake hot water extract enzymatic decomposition product would suppress or accelerate onset.

Female 13-week C57/BL6 mice were given drinking water containing Shiitake hot water extract enzymatic decomposition product and a control group was given sterile water. On day 10 after the ingestion of drinking water, the drinking water was switched to a DSS 1 % aqueous solution. Body weight was then measured once a week and the subsequent survival rate was tracked.

The diminution in body weight on day 10 following the switch to DSS 1 % aqueous solution was 19 % in the control group and 9.5 % in the group given drinking water containing Shiitake hot water extract enzymatic decomposition product. A significant decrease in the group given drinking water containing Shiitake hot water extract enzymatic decomposition product was confirmed. The rate of onset (bloody feces) on day 30 following the switch to DSS 1 % aqueous solution was 100 % in the control group and 17 % in the group given drinking water containing Shiitake hot water extract enzymatic decomposition product. Significant suppression of onset was confirmed in the group given drinking water containing Shiitake hot water extract enzymatic decomposition product.

Based on the above results, it will be understood that the ingestion of drinking containing Shiitake hot water extract enzymatic decomposition decomposed product significantly suppressed inflammatory bowel disease relative to the control group.

### Effect of the Invention

The present invention provides a drug product suited to application in the prevention, improvement in conditions relating to, and treatment of bowel disease, that can be employed as a drug product for intestinal disease, particularly inflammatory bowel disease, has almost no side effects, is highly effective when orally administered, and can be conveniently employed by large numbers of patients. It is highly safe and can be provided in the form of a health food. The present invention further provides a method of preventing, improving conditions relating to, and/or treating bowel disease, and the use of the above-described specific glucan as an active component in such drug products. Accordingly, the present invention can be widely implemented in the fields of medical drugs, food products, medical treatment, feeds, and veterinary drugs, and is thus extremely useful from an industrial perspective.

## Claims

1. A drug product for the prevention, improvement in conditions relating to, and/or treatment of bowel disease, **characterized by** comprising an active component in the form of β (1→3) glucan derived from vegetable material and having a molecular weight of from 5,000 to 20,000.

2. The drug product of claim 1 wherein said vegetable matter is a mushroom.

3. The drug product of claim 1 or 2 wherein said β (1→3) glucan is obtained by hydrolysis of a glucan.

4. The drug product of any of claims 1 to 3 wherein said β (1→3) glucan is obtained from the aqueous extraction product of a mushroom.

5. The drug product of claim 4 wherein said glucan obtained from the aqueous extraction product of a mushroom is obtained by the aqueous extraction - alcohol precipitation of a mushroom or the further decomposition of the precipitate to obtain a compound of low molecular weight.

6. The drug product of any of claims 1 to 4 that is administered orally.

7. The drug product of claim 1 where said bowel disease is inflammatory bowel disease.

8. The drug product of claim 1 where said molecular weight of from 5,000 to 20,000 denotes an average molecular weight of from 5,000 to 20,000.

9. The drug product of claim 2 wherein the type of mushroom is Shiitake.

10. The drug product of claim 1 in the form of a health food product.

11. A method of preventing, improving conditions relating to, and/or treating bowel disease **characterized in that** β (1→3) glucan derived from vegetable material and having a molecular weight of from 5,000 to 20,000 is ingested by or administered to the body.

12. The method of claim 11 wherein the form that is ingested or administered is that of the drug product described in any of claims 1 to 10.

13. The use of β (1→3) glucan derived from vegetable material and having a molecular weight of from 5,000 to 20,000 as a drug product for the prevention, improvement in conditions relating to, and/or treatment of bowel disease.

14. The use of the drug product described in claim 13 in the form of the any of the drug products described in claims 1 to 10.
